# EUROPÄISCHE PATENTSCHRIFT

(11) **EP 1 718 970 B1**
(45) Veröffentlichungstag und Bekanntmachung des Hinweises auf die Patenterteilung: **14.09.2011**
(21) Anmeldenummer: 05707438.7
(22) Anmeldetag: 16.02.2005
(51) Int. Cl.: G01N 33/543

(54) **VERFAHREN UND VORRICHTUNG ZUR BESTIMMUNG MEHRERER ANALYTEN MIT SIMULTANER INTERNEN KONTROLLE IN EINER GRAFISCHEN KOMBINATION**
METHOD AND DEVICE FOR THE DETERMINATION OF SEVERAL ANALYTES WITH SIMULTANEOUS INTERNAL VERIFICATION IN A GRAPHICAL COMBINATION
PROCEDE ET DISPOSITIF POUR DETERMINER PLUSIEURS SUBSTANCES A ANALYSER AVEC CONTROLE INTERNE SIMULTANE EN COMBINAISON GRAPHIQUE

(30) Priorität: 17.02.2004 EP 04003497
(43) Veröffentlichungstag der Anmeldung: 08.11.2006
(60) Teilanmeldung: 10184143.5
(73) Patentinhaber: DST Diagnostische Systeme & Technologien GmbH, 19061 Schwerin (DE)
(72) Erfinder: SCHWERTNER, Heiko, 19055 Schwerin (DE); RUNGE, Dorothee, Monika, 19061 Schwerin (DE)
(74) Vertreter: Simandi, Claus
(86) Internationale Anmeldenummer: PCT/EP2005/001571
(87) Internationale Veröffentlichungsnummer: WO 2005/079135

(56) Entgegenhaltungen:
- EP-A- 0 451 686
- EP-A- 0 505 632
- EP-A- 1 376 131
- EP-A2- 0 119 858
- EP-A2- 1 226 871
- WO-A-94/03774
- WO-A-99/30154
- WO-A1-01/71345
- DE-A1- 19 721 151
- US-A- 4 943 522
- US-A- 5 160 701
- BALDWIN C I ET AL: "Analysis of pigeon intestinal mucin allergens using a novel dot blot assay", CARBOHYDRATE RESEARCH, PERGAMON, GB, vol. 326, no. 1, 1 May 2000 (2000-05-01), pages 43-49, XP004196977, ISSN: 0008-6215, DOI: DOI:10.1016/S0008-6215(00)00023-9
- BLAIS BURTON W ET AL: "Multiplex enzyme immunoassay system for the simultaneous detection of multiple allergens in foods", FOOD CONTROL,, vol. 14, no. 1, 1 January 2003 (2003-01-01), pages 43-47, XP002615635, DOI: DOI:10.1016/S0956-7135(02)00053-1
- BALDWIN C I; CALVERT J E; RENOUF D V; KWOK C; HOUNSELL E F: "Analysis of pigeon intestinal mucin allergens using a novel dot blot assay", CARBOHYDRATE RESEARCH, ELSEVIER SCIENTIFIC PUBLISHING COMPANY, vol. 326, no. 1, May 2000 (2000-05), - May 2000 (2000-05), pages 43-49, XP004196977, AMSTERDAM, NL
- KANBE T; UTSUNOMIYA K; ISHIGURO A: "A crossreactivity at the immunoglobulin E level of the cell wall mannoproteins of Candida albicans with other pathogenic Candida and airborne yeast species", CLINICAL AND EXPERIMENTAL ALLERGY, vol. 27, no. 12, December 1997 (1997-12), - December 1997 (1997-12), pages 1449-1457, ENGLAND
- BLAIS BURTON W; GAUDREAULT MELISSA; PHILLIPPE LUCILLE M: "Multiplex enzyme immunoassay system for the simultaneous detection of multiple allergens in foods", FOOD CONTROL, vol. 14, no. 1, January 2003 (2003-01), - January 2003 (2003-01), pages 43-47,
- "Welweit neuer Allergie-Schnelltest aus Deutschland", RHEIN-ZEITUNG, 21 September 2004 (2004-09-21), Germany Retrieved from the Internet: URL:http://rhein-zeitung.de/on/04/09/22/se rvice/gesundheit/t/rzo89562.html>
- BRAUN T.: "IgE-Test aus der Hüfte", DOCCHEK NEWS, 1 December 2004 (2004-12-01), Retrieved from the Internet: URL:http://news.doccheck.com/de/article/14 1344-ige-test-aus-der-huefte/>
- ZUBERBIER T.: "Screeningverfahren für die Hausarztpraxis: Was kann der neue Allergie-Schnelltest?", MMW FORTSCHRITTE DER MEDIZIN, vol. 146, no. 44, 28 October 2004 (2004-10-28), page 16, XP008103018, Germany
- RICHTER-KUHLMAN E. A.: "Allergieschnelltest: Gewagte Strategie.", DEUTSCHES ÄRZTEBLATT, vol. 101, no. 50, 10 December 2004 (2004-12-10), page A3396, Germany Retrieved from the Internet: URL:http://aerzteblatt.lnsdata.de/pdf/101/ 50/a3396.pdf>
- RICHTER-KUHLMANN E. A.: "Allergien: Schnelle Testung beim Primärarzt", DEUTSCHES ÄRZTEBLATT, vol. 101, no. 42, 15 October 2004 (2004-10-15), page A2777, Germany Retrieved from the Internet: URL:http://www.aerzteblatt.de/v4/archiv/pd f.asp?id=43823>
- DST: 'FastCheckPoc: Instructions for use.' Seiten 1 - 2
- DST: 'FastCheckPoc: Fast allergy test for 2 x 12 allergens' Seiten 1 - 2
- DST: 'FastCheckPoc: Evaluation sheet.' Seite 1

## Beschreibung

Die vorliegende Erfindung betrifft ein Verfahren und eine Vorrichtung zum Allergienachweis von Molekülen oder Molekülklassen oder Molekülgemischen nach anspruch 1-21.

### Hintergrund der Erfindung

In der Forschung, Medizin, Biologie, Chemie, Toxikologie oder einer Vielzahl weiterer Anwendungsfelder stellen analytische Labortests, die zur qualitativen oder/und quantitativen Bestimmung von Molekülen bzw. deren Aktivität oder Zusammensetzung dienen, die Basis für weitreichende Aussagen bis hin zur Entwicklung neuer Verfahren oder Vorrichtungen dar. Beispiel hierfür sind die analytischen Methoden der Molekularbiologie, die zum einen in der Forschung, in forensischen Verfahren zur Aufklärung von Verbrechen oder in der Medizin zur Entdeckung von Krebserkrankungen eingesetzt werden. Die Basis sind die allgemein bekannten Methoden der DNA/RNA Analytik bzw. der Proteinanalytik. Ein weiteres Beispiel sind die Vielzahl von analytischen Verfahren und Methoden, die eingesetzt werden, um die Antikörperreaktionen, sogenannte Immunreaktionen, welche für die Bestimmung von Keimen, Proteinen, Drogen und vielen weiteren Substanzen eingesetzt werden. Diese haben einen der am weitesten gefaßten Anwendungsbereiche. Sie werden sämtlichen Gebieten der Medizin, Forschung, Lebensmitteltechnologie bis zum Bereich der Medikamentation eingesetzt.

Durch diese vielfältigen analytischen Einsatzmöglichkeiten von DNA/RNA, Proteinen und anderen Molekülen sind der Vergangenheit immer weiter die Labortechniken verfeinert worden. Dies führt zu immer größeren Erfolgen, was die Einsatzbereiche wieder erweitert hat. Dies führte in der Konsequenz zu immer größeren Probezahlen, welche gezielt auf bestimmte Moleküle untersucht werden. Da nur mit erheblichem Aufwand eine Vielzahl von einzelnen Proben untersucht werden können werden heute sogenannte Laborroboter eingesetzt oder in neuester Zeit sogenannte Chip-Technologien verwendet, welche in der Lage sind, simultan bis zu mehreren hundert Proben zu untersuchen.

Im Gegensatz zu diesen aufwendigen Laboruntersuchungsmethoden (EP 1 338 895 Titel: High density allergen microarray oder EP 0 875 758 B1 Titel: Immunoassay) werden immer häufiger sogenannte Schnelltests angewandt, welche dem Arzt oder Therapeuten eine schnelle, einfache und auf die wesentlichen Punkte beschränkte analytische Aussage liefern. Der Bedarf ist sehr groß und wächst stetig. Häufig werden diese Tests im sogenannten "Point of Care" Bereich, also unmittelbar vor, während oder nach therapeutischen Maßnahmen, eingesetzt. Ziel ist es auch, eine kostengünstige Alternative zum klassischen Labortest zu bieten. Allerdings ist die Sicherheit der Anwendung oder/und die Zahl der simultan bestimmbaren Analyten sowie der analytischen Genauigkeit, enge Grenzen gesetzt. Hier besteht erheblicher Verbesserungsbedarf.

Es sind Schnelltestverfahren bekannt, wie der Lateral-Flow-Test (LFT), Flow-Through-Test (FTT), Agglutinations-Test (AT) oder Solid-Phase-Test (SPT). Alle diese Verfahren dienen zum schnellen Nachweis von Analyten ohne die Verwendung von Geräten und sind zur visuellen Auswertung geeignet. Alle diese Verfahren haben Nachteile und sind somit nur bedingt Ersatz für Labortests bzw. können nur eingeschränkt durch Laien eingesetzt werden. Deshalb werden diese Verfahren zum Teil durch technische Hilfsmittel ergänzt. Beispiel für diese Verfahren sind u.a. die Patentschriften DE 197 21 151, EP 98 928 264.5, WO 98/53321 Titel: Streifentest zur in vitro Allergiediagnostik; EP 1 369 391; WO96/10747 Titel: Device an method utilizing arrays of structures for analyte capture; WO 03/094716; US 2003-212316 Titel: Method an apparatus for determining blood paramters and vital signs of a patient; WO 02/084249 Titel: Therapeutic an diagnostic uses of antibody specificity profiles. WO 00/40967 Titel: Method and Device for diagnosing allergy Symptoms; WO 02/066602, EP 1350111; WO 93/10458 Binding of Milk allergens to a solid phase; US 6,528,325, WO02/056017 Titel: Method for the visual detection of specific antibodies in human serum by the use of lateral flow assays; EP 1327884 Titel: Reagent test strip comprising conttrol means and timer means; WO 97/31268 Chromatographic strip having detection andcontrol zones oriented paraell to the direction flow; US 6,040,195 Titel: Diagnostic sanitary test strip; US 6,509,196, WO 01/50129 Titel: Compensation for non-spcific signals in quantitative immunoassays.

Die LFT sind die am weitesten verbreiteten Schnelltests. Hier wird die Probe z.B. Serum, Urin, auf eine Fläche aufgetragen. Direkt vergleichbar mit einer Dünnschichtchromatographie wird die Flüssigkeit (Probe) durch eine Trennschicht mit Reagenzien z.B. markierten Antikörpern, weiter durch eine Nitrocelluloseschicht gesogen. In dieser befinden sich Fangschichten, (im Gegensatz zum SPT bei dem sich auf der Oberfläche des Trägers Fangschichten befinden) welche markierte Antikörper binden und eine visuell sichtbare Line bilden. Solche Verfahren sind in der wissenschaftlichen Literatur vor 1980 beschrieben, sowohl in der Anwendung als klassische Dünnschichtchromatographie, als auch als Schnelltest. Unterschiede finden sich nur in der Verwendung eines vereinheitlichten Reaktionsgefäßes oder einer universell und mehrfach verwendbaren Trennschicht.

Der FTT ist vergleichbar einer Säulenchromatographie. Hier fließt die Probe (Flüssigkeit) durch Schichten von Membranen und Saugschichten. Auf der Nachweismembran sind, wie beim LFT z.B.: Antikörper gebunden, welche in der Lage sind Analyten zu binden. Die gebundenen Analyten werden mittels verschiedener Nachweisreagenzien als Punkte sichtbar gemacht. Solche Verfahren sind in der wissenschaftlichen Literatur vor 1980 beschrieben, sowohl in der Anwendung als Säulenchromatographie als auch als Schnelltest. Unterschiede finden sich nur in der Verwendung eines Reaktionsgefäßes oder einer universell und mehrfach verwendbaren Säule.

Das AT-Verfahren basiert auf mit Antikörpern beschichteten Partikeln. Diese befinden sich in einer ebenmäßigen Schicht. Wird eine positive Proben hinzugegeben, so wird die ebenmäßige Schicht durch Quervernetzung zerstört, was bei starken Reaktionen mit bloßem Auge sichtbar ist.

Beim SPT-Verfahren, häufig auch als "dipstick"-Test bezeichnet, werden mittels Antikörpern oder Antigenen die Analyten auf einer Oberfläche gebunden (im Gegensatz zum LPT, bei denen in einer Oberfläche die Analyten gebunden werden) und anschließend durch nachfolgende Reaktionen nachgewiesen. Je nach verwendeter Nachweismethode, kann es zu einem mit bloßem Auge sichtbaren Punkt oder Fläche kommen. Ein solches Verfahren stellt das in der Patentschrift EP 98 928 264.5 (Titel: Streifentest zur in vitro Allergiediagnostik) beschriebene Verfahren dar. Allerdings sind solche Verfahren auch in der wissenschaftlichen Literatur vor 1980 beschrieben.

Alle Verfahren haben individuelle Vor- und Nachteile. Ein idealer Schnelltest sollte mit bloßem Auge auswertbar sein, eine Vielzahl von Analyten in einer Probe bestimmen können, über interne Standards zur Funktions-, Qualitätskontrolle und Mengenbestimmung verfügen und auch für einen Laien auswertbar sein. Ferner sollte der Test schnell, also ca. binnen 25 Minuten oder weniger durchführbar und zugleich kostengünstig herzustellen sein.

LF-Tests können nur einzelne und nur in Ausnahmefällen mehrere Analyten in einer Probe bestimmen, da hier die Probenflüssigkeit durch die Membran fließen muß. Zwar ist hier eine positiv-negativ-Kontrolle möglich, nicht aber deren Quantifizierung. Gleiches gilt für die AT, FTT und SPT Tests, wobei meist auf einen externen Standard wie eine Farbkarte (siehe Patentschrift EP 98928264.5) oder ein Graumuster zurückgegriffen wird. Dies ist für eine visuelle Auswertung, also eine Auswertung mit bloßem Auge, negativ, da es hier leicht zu Fehlinterpretationen kommt. Ferner ist die Empfindlichkeit dadurch stark herabgesetzt. Die, seit den frühen 80'iger Jahren bekannten SPT, AT und FTT Verfahren bieten die Möglichkeit, eine Vielzahl von Analyten in einem Raster (Matrix) vergleichbar den späteren Chip-Technologien im Laborbereich, zu bestimmen. Keines der Verfahren ist jedoch in der Lage, in jedem der Rasterpunkte z.B. mittels eines internen Standards, eine Funktions-, Qualitätskontrolle und Mengenbestimmung zur gewährleisten. Dies ist aber notwendig, da bei einer ungleichmäßigen Verteilung der Probenflüssigkeit über die Tastoberfläche, es zu Konzentrationsunterschieden der zu bestimmenden Analyten über eben dieser Fläche kommt. Dies führt zwangsläufig zu Fehlinterpretationen. Eine Standardreihe mit oder ohne positiv bzw. negativ Kontrolle führt am Rande der Rasterpunkte oder gar durch externe Farbkarte oder Grauabstufung verstärkt diesen Effekt der Fehlinterpretation noch.

WO 94/03774 A betrifft einen Immuno-Assay, wobei eine Auswertung von Analyten mit Hilfe einer Lichtoptik auf dünnen Filmen erfolgt.

WO 99/30154 A offenbart einen Multilayer-Assay, der im Wesentlichen aus einem porösen Material bereitgestellt wird.

Baldwin et al. (BALDWIN C I; CALVERT J E; RENOUF D V; KWOK C; HOUNSELL E F: "Analysis of pigeon intestinal mucin allergens using a novel dot blot assay" CARBOHYDRATE RESEARCH, ELSEVIER SCIENTIFIC PUBLISHING COMPANY, Bd. 326, 43-49 (2000)) beschreibt den Nachweis von Antigenen mit Hilfe sogenannter Dot-Blot-Verfahren, wobei ein Assay auf Hydro-Cellulosebasis verwendet wird.

Kanbe et al. (KANBE T; UTSUNOMIYA K; ISHIGURO A: "A crossreactivity at the immunoglobulin E level of the cell wall mannoproteins of Candida albicans with other pathogenic Candida and airborne yeast species" CLINICAL AND EXPERIMENTAL ALLERGY, Bd. 27, 1449-1457 (1997)) beschreibt einen Allergietest, der ebenfalls als ein Dot-Blot-Verfahren ausgelegt ist.

Blais et al. (BLAIS BURTON W; GAUDREAULT MELISSA; PHILLIPPE LUCILLE M: "Multiplex enzyme immunoassay system for the simultaneous detection of multiple allergens in foods" FOOD CONTROL, Bd. 14, 43-47 (2003)) beschreibt ebenfalls einen Allergietest für Lebensmittelallergie, der ebenfalls auf der Basis eines Dot-Blot-Verfahrens ausgestaltet ist.

EP 0 119 858 betrifft eine Vorrichtung für einen Allergietest, welcher als Bindungs-Assay durchgeführt werden kann.

WO 01/71345 beschreibt ebenfalls eine Vorrichtung zur Durchführung eines Bindungs-Assays, bestehend aus einer Kammer mit einem Einlass und einem Auslass.

All diese vorgenannten Dokumente offenbaren jedoch keine grafische Anordnung, die eine vorteilhafte visuelle Auswertung mehrerer Analyten mit dem bloßen Auge erlaubt.

US 5,160,701 offenbart zwar eine optisch auslesbare grafische Anordnung, jedoch in der Ausführungsform eines LFT-Tests, wie es z. B. zum Schwangerschaftsnachweis aus Urin erforderlich ist. Insbesondere betrifft dieses Dokument kein Koordinatensystem einer grafischen Anordnung für die Auswertung verschiedener Analyten.

Es ist somit Aufgabe der vorliegenden Erfindung, die bestehenden Nachteile der zur Zeit vorherrschenden Vorgehensweise und deren Verfahren aus dem Bereich der Schnelltestverfahren zu vermeiden, ohne die Vorteile der klassischen. Labortestverfahren inkl. Laborroboter und/oder Chip-Systemen zu verlieren. Ferner ist es das Ziel, die Testverfahren so zu vereinfachen, daß diese sogar von Laien durchgeführt werden könnten. Ein weiteres Ziel ist es, die Analysen ohne und/oder nur mit einfachen Hilfsmitteln oder Gerätschaften durchführen zu können.

Diese Aufgabe wird erfindungsgemäß durch ein Verfahren und eine Vorrichtung nach anspruch 1-21 gelöst.

Die in dieser Patentschrift vorgestellte Erfindung ermöglicht somit den Aufbau eines Schnelltestverfahrens, welches universell eingesetzt werden kann, um ein breites Spektrum an Allergene in einer Probe bestimmen zu können. Ferner können parallel ein oder mehrere oder eine Vielzahl an Allergene bestimmt werden. Dies kann in einem beliebigen Raster. (Matrix oder Array) durchgeführt werden, z.B. linear, oder quadratisch. Solche Anordnungen mit Flächen, welche definierte Aufgaben ob aktiv oder passiv erfüllen, finden sich schon in der frühen wissenschaftlichen Literatur aller Fachgebiete und gehören damit zum Stand der Technik; Beispiele sind Dotblot-Arrays, Mikrotiterplatten (MTP) Koordinatensysteme. Erfindungsgemäß werden dabei über interne Standards in jedem Rasterpunkt eine Funktions-, Qualitätskontrolle und/oder Mengenbestimmungen durchgeführt, so daß ein eventueller Konzentrationsgradient der zu bestimmenden Allergene über dem Raster nicht zu Fehlinterpretationen führt. Bei entsprechender Wahl der Nachweisreaktion, z.B. Enzym Immuno Assay (EIA), Nachweis mittels Gold-, Selen-, oder Latexpartikeln gebunden an Antikörpern, kann der Test mit bloßem Auge, also rein visuell ausgewertet werden. Ferner kann der Test mit einfachen Hilfsmitteln sich auch der Nachweisverfahren wie Fluoreszenzmarkierung, elektrochemischer Detektion oder spektroskopischer Verfahren bedienen.

Weiterhin kann über die grafische Gestaltung von Standard zu Probe, eine besondere Auswertungssicherheit gewährleistet werden. Dabei werden allgemein verständliche Symbole verwendet z.B. + für positive Probe (Analyt vorhanden) und - für negative Probe (kein Analyt vorhanden). Für LFT wurde dies in der Patentschrift EP 0 421 294 B1 beschrieben, aber hier kann nur ein Analyt in der Probenflüssigkeit nachgewiesen werden und es ist keine Möglichkeit zur Quantifizierung gegeben. Im erfindungsgemäßen Test können auf diese Weise eine Vielzahl von zu bestimmenden Allergene dargestellt werden und zugleich in jedem Rasterpunkt quantifiziert werden. Ferner können auch andere Darstellungsformen gewählt werden wie Ring und innenliegender Punkt oder Stern.

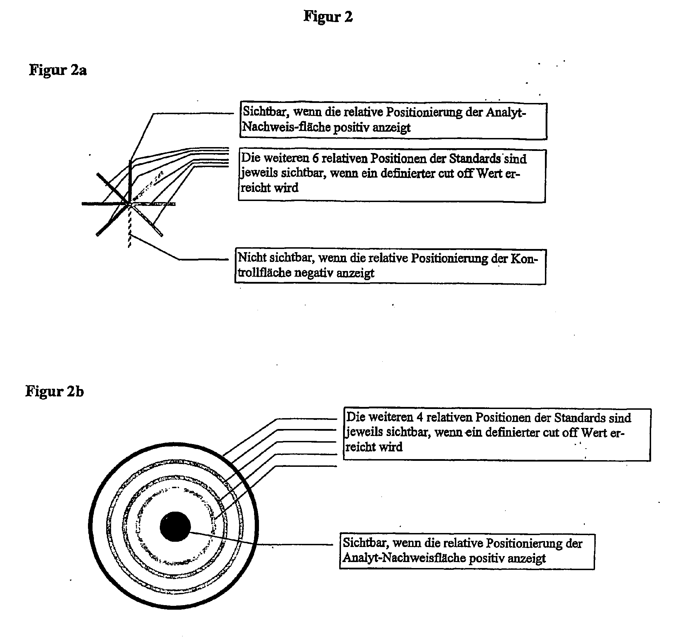

Ein weiteres nicht begrenzendes Beispiel ist ein Sternsymbol (siehe Fig.2). In diesem Falle sind fünf der sechs Sternstrahlen Positiv-Kontrollen (entsprechend einer Standardreihe eines Labortestverfahrens). Diese sind derart eingestellt, daß diese jeweils einzeln ab bestimmten Konzentrationsschwellen (z.B.: aufsteigend) visuell sichtbar werden. Der Allergen wird, falls in der Probe vorhanden, entsprechend seiner Konzentration, ebenfalls sichtbar. Durch die unmittelbare örtliche Zusammenfassung von Meßpunkt (Analytpunkt) und Kontrollen (in diesem Fall einer Standardreihe), wird die sichere Durchführung einer Konzentrationsbestimmung ermöglicht. Die Genauigkeit der visuellen Ablesung ist über die Zahl der Sternstrahlen einstellbar. Die Wahl der grafischen Darstellung hängt von der späteren Anwendung ab. Wird eine reine visuelle, also mit bloßem Auge, auswertbarer Testformat angestrebt, so haben sich die Symbole + -, Kreis mit Punkt oder Stern bewährt. Wird ein Testformat angestrebt, welches mittels technischer Hilfsmittel, gleich welcher Art, auszuwerten ist, so haben sich Kreis mit zentralem Punkt und Quadrat mit zentralem Kreuz bewährt. Insbesondere können bei Auswertung mit technischen Hilfsmitteln, wie CCD-Kamera, Fluoreszenzdetektion, elektrochemischer Detektion oder andere spektroskopische Verfahren auch produktionsmäßige Fehler erkannt und bewertet werden, denn Verschleppung von Testmaterial wird durch Unschärfe zwischen den grafischen Punkten auf einfache Weise erkannt und per Computerauswertung automatisch zu bewerten. Dies ist bei allen Chip-Varianten ob DNA, RNA oder Proteinchips nicht möglich, da kein direkter, enger räumlicher Zusammenhang realisiert wurde. Werden zudem noch Oberflächen zur Immobilisierung von Molekülen eingesetzt, die glatt oder deren Poren kleine bzw. inaktiviert sind, können Probenmaterialien (Probenmatrices) wie Vollblut, Kapillarblut, Serum, Plasma, Urin, Fäzes oder Proben hoher Viskosität und/oder starker Färbung ohne eine weitere Aufbereitungsmethoden direkt in das erfindungsgemäße Testverfahren eingesetzt werden. Ferner können Zellsuspensionen, Gewebebiooptate oder Lösungen aller Art eingesetzt werden.

Die erfindungsgemäße Vorrichtung ist ferner dadurch gekennzeichnet, daß es sich nicht um chromatographische Verfahren, wie den Lateral-Flow-Test (LFT), Flow-Through-Test (FTT) und auch nicht um Verfahrensmethoden wie den Agglutinations-Test (AT) oder Solid-Phase-Test (SPT) handelt, sondern um ein Tangential-Touch-Testprinzip (TTT). Bei dieser Art des erfindungsgemäßen Verfahrens reicht es aus, wenn die zu analysierende Probe mit der Immobilisierungsfläche in Kontakt kommt, dabei ist es unerheblich, ob die Probe fließt, sich in Ruhe befindet oder aktiv und/oder passiv auf die Oberfläche appliziert wird. Das TTT-Prinzip unterscheidet sich insbesondere dadurch, daß es hiermit möglich wird, Meßflächen und Kontrollflächen zeitlich und räumlich simultan mit der Probe in Kontakt zu bringen. So ist z.B. bei allen sogenannt DIP-Stick-Verfahren oder chromatographischen Verfahren nicht möglich, da immer die Benetzung mit der Probe sequentiell erfolgt - ein DIP-Stick wird in die Probeflüssigkeit eingetaucht oder die Probe wir chromatographisch durch Materialien gesogen oder durchflossen. Auch ist es nicht notwendig, die Immobilisierungsflächen derart anzuordnen, daß diese zu einer Fließrichtung optimal ausgerichtet sind (z.B.: EP 0 421 294 B1, Titel: Improved self-performing immunochrommographic device).

Die Kombination von immobilisierten Molekülen auf Oberflächen, gleich welcher Art und Form oder Material, in abgegrenzten Flächen zum Nachweis von Analyten und einer zusätzlichen räumlich eng zugeordneten, grafisch abgestimmten Immobilisierungsfläche oder - Flächen zur Funktionskontrolle und/oder Quantifizierung zu jedem Analytenmesspunkt im Tangential-Touch-Test Prinzip ist bisher nicht beschrieben worden und stellt die erfindungsgemäße Vorrichtung dar.

Die erfindungsgemäße Vorrichtung wird durch ein erfindungsgemäßes Verfahren ergänzt, welches dadurch gekennzeichnet ist, daß der über immobilisierte Moleküle gebundene Analyt mittels einer nachfolgenden Reaktion oder Reaktionen bestimmt werden kann, welche nachfolgend als Nachweisreaktion bzw. Reaktionen bezeichnet werden. Die zur Durchführung benötigten Nachweisreagenzien können sich komplett auf der Oberfläche frei, z.B. als Lyophilisat oder in Lösung befinden oder teilweise bzw. ganz in getrennten Schritten zugegeben werden. Vorteil der Vorrichtung und des Verfahrens ist es, daß alle klassischen Nachweisverfahren, wie Antigen-Antikörperreaktion, markierter Antikörper oder Antigene, Biotin-Avidin, DNA oder RNA-Sonden, elektrochemische Nachweisverfahren oder spektroskopische Verfahren verwendet werden können. Die Kombination dieser Nachweisverfahren mit der erfindungsgemäßen Vorrichtung stellt das erfindungsgemäße Verfahren dar.

Die Zahl und Anordnung der kombinierten Rasterelemente ist nur durch die Anwendung oder den Anwendungszweck limitiert. Bei einer Anwendung hinsichtlich einer ausschließlichen visuellen Auswertung, ist letztlich die Auflösungsgrenze des menschlichen Auges der limitierende Faktor. Bewährt haben sich in diesem Anwendungsfall Rasterformate bis zu einer Zahl von 100. Werden technische Hilfsmittel zur Auswertung hinzugezogen, so können je nach technischem Verfahren und Aufwand bis zu 100.000 Rasterelemente ausgewertet werden.

Im Hinblick auf das oben Gesagte betrifft ein erster Aspekt der vorliegenden Erfindung somit eine Vorrichtung zum Allergienachweis von Molekülen oder Molekülklassen oder Molekülgemischen nach Anspruch 1-7.

Wie oben bereits ausgeführt, ist der vorliegende Test durch die Anordnung der Flächen (oder auch Meßpunkte, Rasterpunkte) in der Lage, in jedem der Rasterpunkte z.B. mittels eines internen Standards, eine Funktions-, Qualitätskontrolle und Mengenbestimmung zur gewährleisten. Dies ist vorteilhaft, da bei einer ungleichmäßigen Verteilung der Probenflüssigkeit über die Testoberfläche zu Konzentrationsunterschieden der zu bestimmenden Analyten über eben dieser Fläche kommt. Dies führt zwangsläufig zu Fehlinterpretationen. Eine Standardreihe mit oder ohne positiv bzw. negativ Kontrolle führt am Rande der Rasterpunkte oder gar durch externe Farbkarte oder Grauabstufung verstärkt diesen Effekt der Fehlinterpretation noch. Beide Flächen sind erfindungsgemäß auf der Oberfläche so aufgebaut und zueinander angeordnet, daß sie gemeinsam ausgewertet werden können, wobei sie durch diesen Aufbau eine optisch auslesbare grafische Anordnung bilden. Bevorzugt ist somit eine erfindungsgemäße Vorrichtung zum Nachweis von Molekülen oder Molekülklassen oder Molekülgemischen die dadurch gekennzeichnet ist, daß die Flächen planar und/oder räumlich zueinander angeordnet sind. Weiter bevorzugt sind die Flächen erfindungsgemäß auf der Oberfläche so aufgebaut und zueinander angeordnet, daß sie gemeinsam visuell ausgewertet werden können. Solch eine visuelle Auswertung erfolgt zum Beispiel durch räumlich eng zugeordneten, grafisch abgestimmte Flächen.

Erfindungsgemäß sind die beiden Flächen so aufgebaut, daß sie im wesentlichen zum gleichen Zeitpunkt mit einer gesamten Probenmatrix, aus der Molekülen oder Molekülklassen oder Molekülgemischen bestimmt werden sollen, in Kontakt kommen. Dies wird erfindungsgemäß als "Tangential-Touch-Test Prinzip" verstanden. Bei dieser Art des erfindungsgemäßen Verfahrens reicht es zudem aus, wenn die zu analysierende Probe mit der Immobilisierungsfläche in Kontakt kommt, dabei ist es unerheblich, ob die Probe fließt, sich in Ruhe befindet oder aktiv und/oder passiv auf die Oberfläche appliziert wird. Das TTT-Prinzip unterscheidet sich somit insbesondere von anderen Verfahren dadurch, daß es hiermit möglich wird, Meßflächen und Kontrollflächen zeitlich und räumlich simultan mit der Probe in Kontakt zubringen.

Erfindungsgemäß kann die Probenmatrix, aus der die oder der Allergen bestimmt werden soll, flüssig, fest, gasförmig oder in physikalischen Zwischenzuständen oder Gemischen davon vorliegen.

In einem weiteren Aspekt der erfindungsgemäßen Vorrichtung zum Allergienachweis von Molekülen oder Molekülklassen oder Molekülgemischen ist diese dadurch gekennzeichnet, daß die Flächen in einem oder einer Vielzahl von Symbolen linear oder in einer anders angeordneten Matrix dargestellt werden.

In einem noch weiteren Aspekt der erfindungsgemäßen Vorrichtung zum Allergienachweis von Molekülen oder Molekülklassen oder Molekülgemischen ist diese dadurch gekennzeichnet, daß die immobilisierten Moleküle oder Molekülklassen und/oder Gemische mittels einer Nachweisreaktion visuell ohne weitere technische Hilfsmittel bestimmt werden können, wobei die verschieden Flächen farbig, schwarz oder grau getönt oder einem Gemisch aus Farben und/oder Grautönen erscheinen.

Bevorzugt ist eine erfindungsgemäße Vorrichtung zum Allergienachweis von Molekülen oder Molekülklassen oder Molekülgemischen, die als Gefäß mit einer oder mehreren Öffnungen ausgestaltet ist. Weiter bevorzugt ist eine erfindungsgemäße Vorrichtung zum Nachweis von Molekülen oder Molekülklassen oder Molekülgemischen nach die dadurch gekennzeichnet ist, daß sich die Flächen im Innenraum des Gefäßes befinden oder sich eine oder mehrere Flächen auf der Gefäßwandung befindet

In einem noch weiteren Aspekt der erfindungsgemäßen Vorrichtung zum Allergienachweis von Molekülen oder Molekülklassen oder Molekülgemischen ist diese dadurch gekennzeichnet, daß als Symbolpaare - für negativ und + für positiv, oder Kreis für negativ und Kreis mit innen liegendem Punkt/Punkten für positiv sichtbar werden.

Gemäß einem weiteren Aspekt der erfindungsgemäßen Vorrichtung zum Allergienachweis von Molekülen oder Molekülklassen oder Molekülgemischen ist diese dadurch gekennzeichnet, daß die Mittel zur Immobilisierung von Molekülen oder Molekülklassen und/oder Gemischen ausgewählt sind aus der Gruppe bestehend aus Antikörper, Antigenen, DNA, RNA, Enzymen, Substraten, Rezeptoren, Liganden und Kombinationen davon.

Die Aufgabe der vorliegenden Erfindung wird weiterhin durch ein Verfahren zum Allergienachweis von Molekülen oder Molekülklassen oder Molekülgemischen gelöst nach anspruch 8-21.

Gemäß einem weiteren Aspekt des Verfahrens der Erfindung ist dieses dadurch gekennzeichnet, daß die verschieden Nachweisflächen farbig, schwarz oder grau getönt oder einem Gemisch aus Farben und/oder Grautönen erscheinen. Bevorzugt ist, daß die Flächen in einem oder einer Vielzahl von Symbolen linear oder in einer anders angeordneten Matrix ausgelesen werden. Beispiele für solche Anordnungen sind rasterförmige oder schachbrettartige Anordnungen oder Anordnung in einem Kreis, anderen grafischem Symbol oder Buchstaben oder Zahlen. Weiter bevorzugt ist, daß als Symbole - für "negativ" und + für "positiv", oder Kreis für "negativ" und Kreis mit innen liegendem Punkt/Punkten für "positiv" sichtbar werden.

Gemäß eines noch weiteren Aspekts des Verfahrens der Erfindung ist dieses dadurch gekennzeichnet, daß Symbole aus mehreren ineinander liegenden Kreisen mit einem zentralen Punkt sichtbar werden, der nur im positiven Nachweisfall erscheint, und wobei jeder einzelne Kreis nur ab einem bestimmten Konzentrationswert des Analyten erscheint oder ein Stern, bei dem jeder der Strahlen ab einem bestimmten Konzentrationswert sichtbar wird, sowie im positiven Fall ein zuvor definierter Strahl erscheint oder das die einzelnen Strahlen verschiedene Analyten nachweisen und ein Strahl an einem bestimmten Konzentrationswert erscheint oder einer Kombination aus diesen Symbolpaaren.

Gemäß eines noch weiteren Aspekts des Verfahrens der Erfindung ist dieses dadurch gekennzeichnet, daß die Probenmatrix, aus der die oder der Analyt bestimmt wird, flüssig, fest, gasförmig oder in physikalischen Zwischenzuständen oder Gemischen davon vorliegt Bevorzugt ist, daß als Probenmatrix Vollblut, Kapillarblut, Nabelschnurblut, arterielles oder venöses Vollblut, Serum, Plasma, Urin, Fäzes, Tränenflüssigkeit, Speichel, Körperschleim, gefärbte Lösungen, Lösungen mit festen Bestandteilen oder hochviskose Flüssigkeiten verwendet werden.

Weiter bevorzugt ist, daß die Probe vor, während oder geschaltet mittels Aufreinigung, Aliquotierung, Derivatisierung und/oder Isolation zur Auftragung auf die erfindungsgemäße Oberfläche vorbereitet wird.

Gemäß eines noch weiteren Aspekts des Verfahrens der Erfindung ist dieses dadurch gekennzeichnet, daß die Nachweisreaktionen von Molekülen oder Molekülklassen oder Molekülgemischen ausgewählt sind aus Farbstoff-, Radionukleotid-, Antikörper-, DNA- oder RNA-, Biotin-, Avidin- oder Enzym-Nachweisreaktionen oder Kombinationen davon.

Bevorzugt ist ein Verfahren der Erfindung" bei dem die immobilisierten Moleküle oder Molekülklassen und/oder Gemische mittels einer Nachweisreaktion visuell ohne weitere technische Hilfsmittel bestimmt werden. Jedoch kann ein alternatives Verfahren der Erfindung dadurch gekennzeichnet sein, daß für die Auslesung und/oder Auswertung technische Hilfsmittel verwendet werden, um eine visuelle Auswertung zu ermöglichen oder das Verfahren, wie zum Beispiel densitometrische Verfahren, spektroskopische - oder elektrochemische Verfahren mit der erfindungsgemäßen Auslesung und/oder Auswertung kombiniert werden. Auch kann das Verfahren der Erfindung dadurch gekennzeichnet sein, daß dieses mit Flow-Through-Tests, Agglutinations-Tests und/oder mit Solid-Phase-Tests kombiniert wird und eines, mehrere oder viele Symbolpaare umfaßt Auch kann das Verfahren der Erfindung dadurch gekennzeichnet sein, daß dieses mit Schnelltestverfahren Lateral-Flow-Test kombiniert wird und mindestens zwei, mehrere oder viele Symbolpaare umfaßt.

Im Rahmen der vorliegende Erfindung sollen die folgenden Begriffe bedeuten (Liste der Definitionen sofern diese nicht schon international festgelegt sind. Bei nicht im folgenden aufgelisteten Abkürzungen werden immer die international gebräuchlichen bzw. festgelegten Standards verwendet)
- Aliquotierung: Aufteilung von Flüssigkeiten in kleinere Mengeneinheiten bzw. Volumina, z.B. Entnahme einer kleineren Menge aus einer Pro- be.
- Analyt: Nachzuweisendes Molekül oder Molekülgruppe
- AT: Agglutinations-Test
- Aufreinigung: Abtrennung von Flüssigkeiten, Partikeln, Substanzklassen, Zel- len oder Zellbestandteilen, Verunreinigungen.
- Derivatisierung: Chemische oder physikalische Modifikation einer Substanz oder Substanzklasse
- FTT: Flow-Through-Test
- Isolierung: Trennung einer Substanz oder Substanzklasse aus einem kom- plexen Gemisch, gleich ob sich dieses in einer festen, gasförmi- gen oder flüssigen Phase oder einer Kombination befindet.
- LFT: Lateral-Flow-Test
- Nachweis: Bestimmung des Vorhandensein eines oder mehrerer des Mole- küle oder Molekülgruppen mit oder ohne Quantifizierung
- Parameter: Synonym zu Analyt
- Quantifizierung: Mengenmäßige Bestimmung von Molekülen oder Molekülgrup- pen
- SPT: Solid-Phase-Test
- Technische Hilfsmittel: Unter technische Hilfsmittel werden alle einfachen Mittel ver- standen, die zur Sichtbarmachung, Verstärkung, Modifikation, Auswertung oder deren Kombination verwendet werden. Z.B. Folien, Lichtquellen, Detektoren aller Art, chemische oder phy- sikalische Reaktionen, welche vor während oder nach der Mes- sung angewendet werden. Eine Brille welche nur zur Verbesse- rung des natürlichen Seevermögens dient, wird nicht als techni- sches Hilfsmittel bezeichnet.
- Visuelle Auswertung: Beurteilung eines Messergebnisses mittels ausschließlicher in Augenscheinnahme oder auch mit bloßem Auge. Dabei zählt ei- ne Brille nicht als technisches Hilfsmittel
- Negativ: Analyt ist nicht in der Probe oder unter einem festgelegten cut- off Wert.
- Positiv: Analyt ist in der Probe und/oder über einem bestimmten festge- legt cut-off Wert.
- Matrix: Synomym eng.: Array Beliebige Anordnung und Anzahl von Flächen, welche innerhalb eines Koordinatensystems individuell identifizierbar sind, z.B.: eines klassischen Schachbrettkoordina- tensystems oder MTP Koordinatensystems.
- MTP: Mikrotiterplatten
- Cut off: Konzentrationsgrenzwert ab den das Ergebnis bei vorhandenem Analyt als positiv, im Sinne einer Ergebnisbeurteilung, zu wer- ten ist.

Das erfindungsgemäße Verfahren kann in voneinander abweichenden alternativen technischen Vorgehensweisen durchgeführt werden und in der Kombination mit klassischen Schnelltestverfahren angewendet werden.

Die Erfindung soll in den nachfolgenden Beispielen unter bezug auf die beigefügten Zeichnungen weiter erläutert werden. Die Beispiele sollen den Umfang der Erfindung nicht einschränken, sondern die Möglichkeiten des Verfahrens aufzeigen.

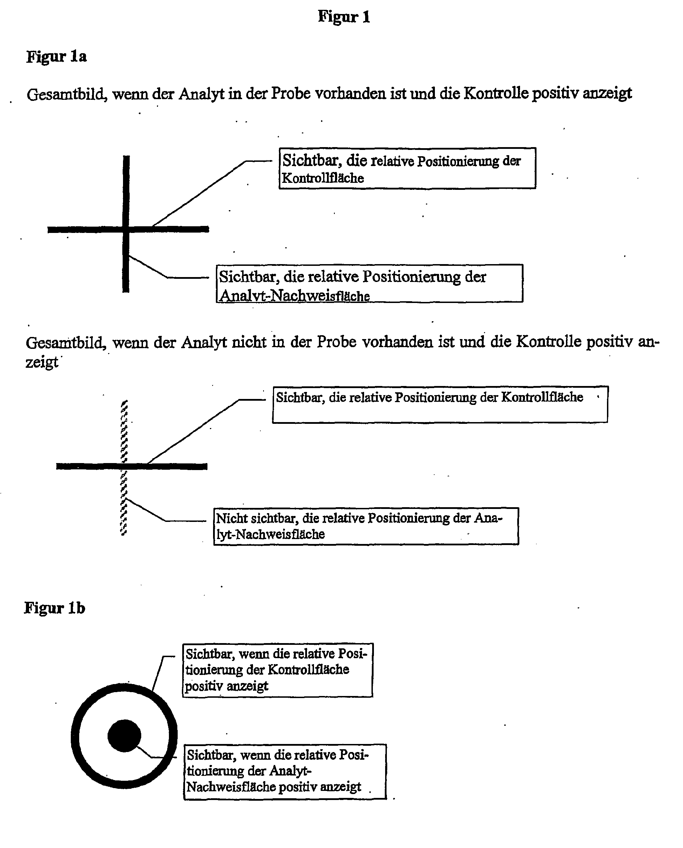

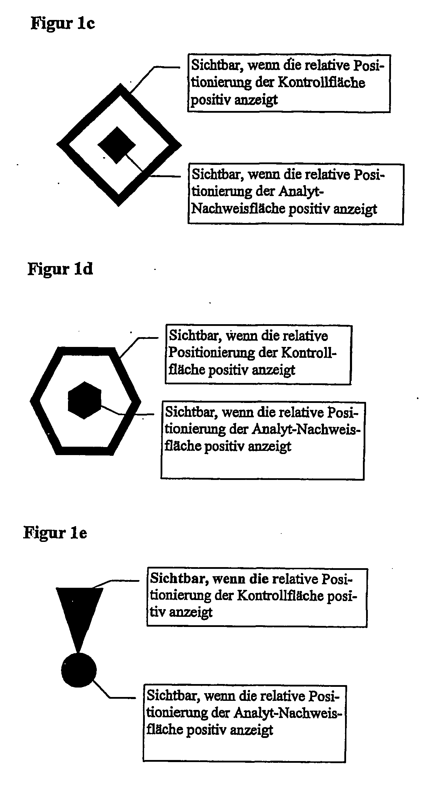

Fig. 1 zeigt Beispiele für grafische Symbolpaare mit einfacher Positiv-Kontrolle eines definierten cut off - Fig. 1a - Symbol "Kreuz"; Fig. 1b - Symbol "Kreis mit Punkt"; Fig. 1c - Symbol "Raute mit innerer Raute"; Fig. 1d - Symbol "Vieleck mit Vieleckzentrum"; und Fig. 1e - Symbol "Dreiecke mit Punkt"
Fig. 2 zeigt Beispiele für grafische Symbolpaare mit stufenweiser Standardreihe mit oder ohne Negativ-Kontrolle - Fig. 2a - Symbol "Stern mit Negativ-Kontrolle"; Fig. 2b - Symbol "Kreise ohne Negativ-Kontrolle";

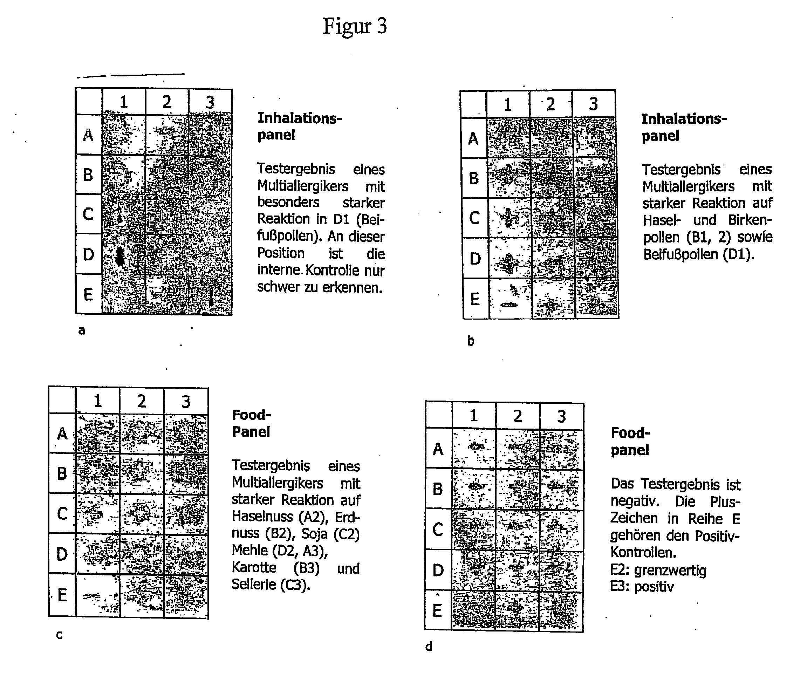

Fig. 3 zeigt Beispiele für erfindungsgemäße Ausführungsformen von Anordnungen anhand zweier Inhalationspanels (Figur 3 a, b) und zweier Nahrungsmittel("Food")-panels (Figur 3 c, d).

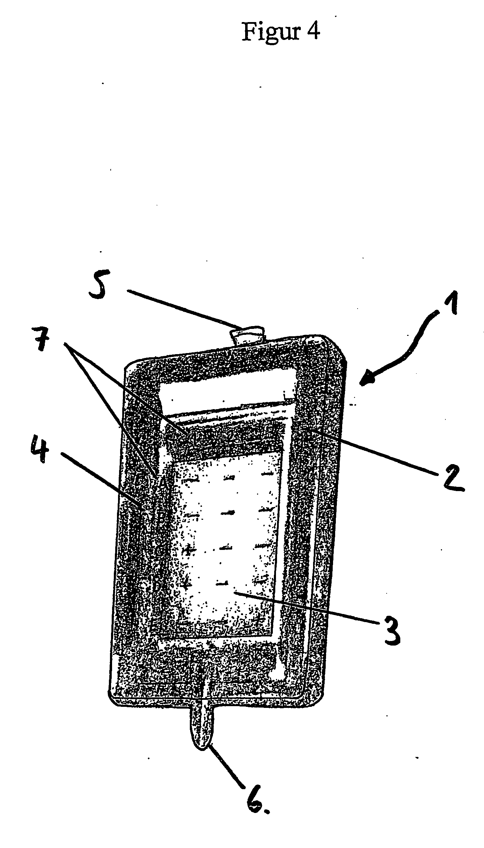

Fig. 4 zeigt ein Beispiel einer erfindungsgemäßen Ausführungsform einer Vorrichtung (1) aus Beispiel 7, mit einer Kammer (2), Membran (3), Kammerdeckel (4), obere Öffnung der Kammer (5), untere Öffnung der Kammer (6) und einem Koordinatensystem (7).

### Beispiele

Das erfindungsgemäße Verfahren kann mit dem bekannten Schnelltestverfahren wie dem Lateral-Flow-Test (LFT), Flow-Through-Test (FTT), Agglutinations-Test (AT) oder Solid-Phase-Test (SPT) kombiniert werden. Ferner können alle optischen Verfahren, wie densitometrische Verfahren, spektroskopische - oder elektrochemische Verfahren mit dem erfindungsgemäßen Verfahren kombiniert werden. Beispiele verfahrenstechnischer Kombinationen sind:
1. Zur Sichtbarmachung von grafisch direkt verbundenen Symbolen nach dem erfindungsgemäßen verfahren, gleich welcher Empfindlichkeitsstufe (Visuell oder mit technischen Hilfsmitteln), können Antikörper/Antigen-Reaktionen, DNA- oder RNA-Sondenreaktionen, Enzym/Substratreaktionen, Rezeptor/Ligand-Reaktionen oder Oberflächenaffinitätsreaktionen oder deren Kombinationen verwendet werden. Die Auswahl der Nachweisreaktion ist nur abhängig von dem oder den nachzuweisenden Analyten und den Bedingungen unter denen der Nachweis stattfinden soll.
2. Durchführung des Verfahrens mit der erfindungsgemäßen Vorrichtung mit einem speziellen Gefäß oder ohne ein solches.
3. Durchführung des Verfahrens mit einer zuvor, während oder nachgeschalteten durchgeführten Aufreinigung, Aliquotierung, Derivatisierung und/oder Isolation.

### Beispiel 7 - Beispiel für eine erfindungsgemäße Vorrichtung und deren Verwendung

Ein Beispiel einer erfindungsgemäße TTT-Vorrichtung und deren Verwendung betrifft einen Allergie-Screeningtest auf 12 Lebensmittelallergene durch den Arzt mit zwei Tropfen Blut. Es können auch jeweils 100 µl EDTA-Blut, Heparin-Blut oder Serum verwendet werden. Dieser Test erlaubt eine schnelle Übersicht, auf welche Allergene der Patient reagiert. Ein quantitativer Bestätigungstest sowie Testung auf weitere Allergene kann im Anschluss durchgeführt werden. Das Verfahren gliedert sich grob in a) Blutentnahme (Inkubationszeit des Blutes: 15 Min.); b) Waschvorgang (Inkubationszeit des Substrates: 5-10 Min); c) zweiter waschvorgang; d) Ablesung; und e) Auswertung.

### Packungsinhalt der FastCheckPOC® erfindungsgemäßen Vorrichtung

- 50 ml Waschlösung, 300 µl Testlösung, 3 ml Farbsubstrat
- 1 x Pipette, 1 x Lanzette, 1 x Kapillarröhrchen, 1 x 10 ml-Plastikspritze, 1 x 5 ml-Plastikspritze
- 1 x Filtereinheit mit 12 Allergenen und 3 Kontrollen
- Informationen zur Testauswertung

### Vorbereitungen/Blutentnahme

1. Entnahme des Kapillarröhrchens, des Schraubdeckelröhrchens sowie der Lanzette aus der Verpackung.
2. Öffnen des braunen 2 ml Schraubdeckeköhrchens mit der **Testlösung** und Aufstellen auf eine gerade Unterlage.
3. Blutentnahme, z.B. aus der Fingerkuppe z.B. mittels der Lanzette. Alternativ können auch 100 µl EDTABlut, Heparin-Blut oder Serum eingesetzt werden.
4. Entnehmen der Kapillare aus dem klaren Röhrchen und öffnen des Deckels. Aufnehmen von zwei vollen Tropfen Blut mit der Kapillare Hinzugeben des Blutes in das Schraubdeckelröhrchen mit der Testlösung. Dadurch wird das Blut in die Testlösung transferiert.

### Testdurchführung

1. Öffnen der Flasche mit der **Waschlösung** und die Kammer mit dem Testfilter und Befeuchten des Testfilters mit ein wenig Waschlösung an. Zur gleichmäßigen Verteilung der Flüssigkeit sollte die Einmalpipette verwendet werden.
2. Aufsaugen der gesamten Blut-Testlösung mit der Einmalpipette. Auftragen dieser Lösung auf den **Filter** in der Testkammer und Verteilen der Lösung mit Hilfe der Einmalpipette auf der Membran.
3. Schließen der Kammer zunächst nur soweit, bis die kleine Lasche an der rechten Seite der Testkammer geschlossen werden kann. Ein Ausflocken des Blutes beeinträchtigt nicht das Ergebnis, Es sollte jedoch kein Blut am Kammerdeckel zurückbleiben, da sonst der Filter nicht ausreichend getränkt wird. Die **Inkubationszeit** beträgt **15 Minuten.**
4. Vollständiges Schließen der Testkammer. Nach dem kompletten Schließen ist die Kammer jetzt nicht mehr zu öffnen. Entfernen des Verschlussdeckels von der oberen Kammeröffnung.
5. Im ersten Waschschritt wird die Testkammer zunächst mit **Leitungswasser** gespült. Dazu wird die Spritze (10 ml) an der oberen Öffnung der Kammer fixiert und das Wasser gleichmäßig in die Kammer gedrückt Halten der Kammer mit der unteren Öffnung über ein Waschbecken oder Gefäß, in das die Flüssigkeit ablaufen kann. Wiederholen des Vorgangs 2 bis 3mal. Der Filter darf eine rosa Färbung aufweisen. Aufziehen der **Waschlösung** mit der Spritze (10 ml) und Injizieren dieser durch langsames und stetiges Drücken durch die obere Kammeröffnung in das Gehäuse. Ein Teil der Waschlösung verbleibt für **2 Minuten** in der Kammer, erst danach wird die Spritze von der Öffnung entfernt, so daß die Flüssigkeit vollständig ablaufen kann. Wiederholen des Vorgangs weitere **2mal.** Danach wird der Testfilter 2mal mit jeweils 10 ml Waschlösung durchgespült. Ablaufen der Flüssigkeit
6. Aufziehen der kleinen 5 ml-Spritze mit **Substrat** (schwarze 3 ml-Flasche) und verbinden dieser mit der Kammer an der oberen, großen Öffnung, wobei das Gehäuse waagerecht gehalten wird, um Luftblasen zu vermeiden. Einspritzen des Substrats gleichmäßig in die Kammer.
7. Legen der Testkammer flach auf eine ebene Unterlage. Die Kammer muss waagerecht liegen und die Spritze sollte auf der Kammeröffnung verbleiben, um ein Austreten des Substrates zu vermeiden. **Inkubieren** das Substrats **5-10 Minuten** in der Kammer.
8. Entfernen der Spritze und ablaufen lassen der Flüssigkeit aus der Kammer.
9. Aufziehen von Leitungswasser mit der großen Plastikspritze (10 ml) auf und verbinden dieser mit der oberen Öffnung der Kammer.
10. **Spülen** der Membran mit dem Inhalt der Spritze, indem die Kammer mit der zweiten, kleineren Öffnung nach unten über ein Waschbecken oder ein Gefäß gehalten wird, in das die Spülflüssigkeit abfließen kann. Wiederholen des Spülvorgang jedoch sollte nun etwas Wasser in der Kammer verbleiben, um die Ablesung zu erleichtern. Die Auswertung sollte innerhalb von 10 Minuten erfolgen, solange der Filter noch feucht ist! Bei längerer Wartezeit kann es zu unspezifischen Nachreaktionen kommen, die das Ergebnis verfälschen.

### Auswertung

1. Auf dem Testfilter befinden sich fünf Reihen in drei Spalten. Die untere Reihe enthält von links nach rechts eine Negativ-, eine Grenzwert- und eine Positivkontrolle, die restlichen vier Reihen enthalten die Allergene.
2. Auswerten Sie die Resultate anhand des Koordinatensystems aus und übertragen dieser in den Auswertungsbogen.
3. Ein Minus-Zeichen bedeutet, dass der Test an dieser Stelle negativ ist, es liegt keine Allergie vor. Ein Pluszeichen bedeutet eine positive Reaktion, es liegt spezifisches IgE gegen das entsprechende Allergen vor. Die Position der Allergene ist auf dem Auswertungsbogen vorgegeben.
4. Markieren auf dem Auswertungsbogen des Ergebnisses in der dafür vorgesehenen Spalte.

### Liste der zitierten Patentliteratur

- DE 19721151, EP 98928264.5, WO 98/53321 Titel: Streifentest zur in vitro Allergiediagnostik.
- EP 0421 294 B1 Titel: Improved self-performing immunochromatographic device.
- EP 1369391; WO96/10747 Titel: Device an method utilizing arrays of structures for analyte capture.
- WO 03/094716; US 2003-212316 Titel: Method an apparatus for determining blood paramters and vital signs of a patient.
- WO 02/084249 Titel: Therapeutic an diagnostic uses of antibody specificity profiles.
- WO 00/40967 Titel: Method and Device for diagnosing allergy Symptoms
- EP 0875758 B1 Immunoassay.
- WO 02/066602, EP 1350111 WO 93/10458Titel: Binding of Milk allergens to a solid phase.
- EP 1338895 Titel: High density allergen microarray
- US 6,528,325, WO02/05601 Titel: Method for the visual detection of specific antibodies in human serum by the use of lateral flow assays.
- EP 1327884 Titel: Reagent test strip comprising conttrol means and timer means
- WO 97/31268 Titel: Chromatographic strip having detection andcontrol zones oriented paraell to the direction flow
- US 6,040,195 Titel: Diagnostic sanitary test strip.
- US 6,509,196, WO 01/50129 Titel: Compensation for non-spcific signals in quantitative immunoassays.

## Patentansprüche

1. Vorrichtung zum Allergienachweis von Molekülen oder Molekülklassen oder Molekülgemischen, **dadurch gekennzeichnet, daß**
a) auf einer Oberfläche der Vorrichtung mindestens zwei Flächen mit immobilisierten Molekülen oder Molekülklassen versehen sind, wobei eine Fläche zur Kontrolle bzw. Standardisierung, und die andere Fläche zum Nachweis eines Analyten verwendet wird, wobei
b) die beiden Flächen so aufgebaut sind, daß diese beiden Flächen zum gleichen Zeitpunkt mit einer gesamten Blutprobe, aus der Allergene bestimmt werden sollen, in Kontakt kommen, und
c) wobei beide Flächen so aufgebaut und zueinander angeordnet sind, daß diese beiden Flächen gemeinsam ausgewertet werden und ein Gesamtbild einer optisch auslesbaren graphischen Anordnung bilden, und
d) wobei die Flächen in einem Koordinatensystem oder Matrix angeordnet sind und mit dem bloßen Auge auswertbar sind, und
e.) wobei diese Vorrichtung als Gefäß ausgestaltet ist,
f.) wobei die Flächen in einer Vielzahl von Symbolen linear, oder in einer anders angeordneten Matrix dargestellt werden.

2. Vorrichtung zum Allergienachweis von Molekülen oder Molekülklassen oder Molekülgemischen nach Anspruch 1, **dadurch gekennzeichnet, daß** die Flächen planar und/oder räumlich zueinander angeordnet sind.

3. Vorrichtung zum Allergienachweis von Molekülen oder Molekülklassen oder Molekülgemischen nach Anspruch 1 oder 2, **dadurch gekennzeichnet, daß** die Probe, aus der die oder der Analyt bestimmt werden soll flüssig, fest, gasförmig oder in physikalischen Zwischenzuständen oder Gemischen davon vorliegt.

4. Vorrichtung zum Allergienachweis von Molekülen oder Molekülklassen oder Molekülgemischen nach einem der Ansprüche 1 bis 3 **dadurch gekennzeichnet, daß** die immobilisierten Moleküle oder Molekülklassen mittels einer Nachweisreaktion visuell ohne weitere technische Hilfsmittel gemeinsam ausgewertet werden, wobei die verschieden Flächen farbig, schwarz oder grau getönt oder einem Gemisch aus Farben und/oder Grautönen erscheinen.

5. Vorrichtung zum Allergienachweis von Molekülen oder Molekülklassen oder Molekülgemischen nach einem der Ansprüche 1 bis 4, **dadurch gekennzeichnet, daß** sich die Flächen im Innenraum des Gefäßes befinden oder sich eine oder mehrere Flächen auf der Gefäßwandung befindet.

6. Vorrichtung zum Allergienachweis von Molekülen oder Molekülklassen oder Molekülgemischen nach einem der Ansprüche 1 bis 5, **dadurch gekennzeichnet, daß** das Gesamtbild einer optisch auslesbaren graphischen Anordnung als Symbole-für negativ und + für positiv, oder Kreis für negativ und Kreis mit innen liegendem Punkt/Punkten für positiv sichtbar werden.

7. Vorrichtung zum Allergienachweis von Molekülen oder Molekülklassen oder Molekülgemischen nach einem der Ansprüche 1 bis 6, **dadurch gekennzeichnet, daß** die immobilisierten Moleküle oder Molekülklasse und/oder Gemische ausgewählt sind aus der Gruppe bestehend aus Antikörpern. Antigenen, Allergene, DNA, RNA, Enzymen, Substraten, Rezeptoren, Liganden und Kombinationen davon.

8. Verfahren zum Allergienachweis von Molekülen oder Molekülklassen oder Molekülgemischen, umfassend
a) ein Kontakt bringen einer Blutprobe, aus der Allergene bestimmt werden sollen, mit der Oberfläche einer Vorrichtung auf solche Weise, daß diese zum gleichen Zeitpunkt mit der gesamten Blutprobe in Kontakt kommt, aus der Allergene bestimmt werden sollen, wobei auf der Oberfläche der Vorrichtung mindestens zwei Flächen derart mit immobilisierten Molekülen oder Molekülklassen und/oder Gemischen versehen sind, daß eine Fläche zur Kontrolle bzw. Standardisierung, und die andere zum Nachweis eines Analyten vorgesehen ist und wobei beide Flächen so aufgebaut und zueinander angeordnet sind, daß die beiden Flächen gemeinsam ausgewertet werden und ein Gesamtbild einer optisch auslesbaren graphischen Anordnung bilden, und
b) Auslesung und Auswertung der Flächen anhand eines Koordinatensystems oder Matrix;
wobei diese Vorrichtung als Gefäß ausgestaltet ist, und die Flächen in einer Vielzahl von Symbolen linear, oder in einer anders geordneten Matrix dargestellt werden.

9. Verfahren nach Anspruch 8, **dadurch gekennzeichnet, daß** die Auslesung der Flächen planar und/oder räumlich erfolgt.

10. Verfahren nach einem der Ansprüche 8 oder 9, **dadurch gekennzeichnet, daß** die verschiedenen Nachweisflächen farbig, schwarz oder grau getönt oder einem Gemisch aus Farben und/oder Grautönen erscheinen.

11. Verfahren nach einem der Ansprüche 8 bis 10, **dadurch gekennzeichnet, daß** die Flächen in einem oder einer Vielzahl von Symbolen linear oder in einer anders angeordneten Matrix ausgelesen werden.

12. Verfahren nach einem der Ansprüche 8 bis 11, **dadurch gekennzeichnet, daß** das Gesamtbild einer optisch auslesbaren graphischen Anordnung als Symbole "-" für negativ und "+" für positiv, oder Kreis für negativ und Kreis mit innen liegendem Punkt/Punkten für positiv sichtbar werden.

13. Verfahren nach Anspruch 12, **dadurch gekennzeichnet, daß** als Symbole mehrere ineinander liegende Kreise mit einem zentralen Punkt sichtbar werden, der nur im positiven Nachweisfall erscheint, und wobei jeder einzelne Kreis nur ab einem bestimmten Konzentrationswert des Analyten erscheint oder ein Stern, bei dem jeder der Strahlen ab einem bestimmten Konzentrationswert sichtbar wird, sowie im positiven Fall ein zuvor definierter Strahl erscheint oder das die einzelnen Strahlen verschiedene Analyten nachweisen und ein Strahl an einem bestimmten Konzentrationswert erscheint oder einer Kombination aus diesen Symbolen.

14. Verfahren nach einem der Ansprüche 8 bis 13, **dadurch gekennzeichnet, daß** die Blutprobe, aus der die oder der Analyt bestimmt wird, flüssig, fest, gasförmig oder in physikalischen Zwischenzuständen oder Gemischen davon vorliegt.

15. Verfahren nach einem der Ansprüche 8 bis 14, **dadurch gekennzeichnet, daß** als Blutprobe Vollblut, Kapillarblut, Nabelschnurblut, arterielles oder venöses Vollblut, Blutserum oder Blutplasma verwendet werden.

16. Verfahren nach einem der Ansprüche 8 bis 15, **dadurch gekennzeichnet, daß** die Blutprobe vor, während oder dazwischenliegend mittels Aufreinigung, Aliquotierung, Derivatisierung und/oder Isolation zur Auftragung auf die Oberfläche der erfindungsgemäßen Vorrichtung vorbereitet wird.

17. Verfahren nach einem der Ansprüche 8 bis 16, **dadurch gekennzeichnet, daß** die Nachweisreaktionen von Molekülen oder Molekülklassen oder Molekülgemischen ausgewählt sind aus Farbstoff-, Radionukleotid-, Antikörper-, DNA-oder RNA-, Biotin-, Avidin-oder Enzym-Nachweisreaktionen oder Kombinationen davon.

18. Verfahren nach einem der Ansprüche 8 bis 17, **dadurch gekennzeichnet, daß** die immobilisierten Moleküle oder Molekülklassen und/oder Gemische mittels einer Nachweisreaktion visuell ohne weitere technische Hilfsmittel bestimmt werden.

19. Verfahren nach einem der Ansprüche 8 bis 18, **dadurch gekennzeichnet, daß** für die Auslesung und/oder Auswertung technische Hilfsmittel verwendet werden, um eine visuelle Auswertung zu ermöglichen oder das Verfahren mit der erfindungsgemäßen Auslesung und/oder Auswertung kombiniert werden, wie zum Beispiel densitometrische Verfahren, spektroskopische oder elektrochemische Verfahren.

20. Verfahren nach einem der Ansprüche 8 bis 19, **dadurch gekennzeichnet, daß** das Verfahren mit Flow-Through-Tests, Agglutinations-Tests und/oder mit Solid-Phase-Tests kombiniert wird und eines, mehrere oder viele Symbole umfasst.

21. Verfahren nach einem der Ansprüche 8 bis 20, **dadurch gekennzeichnet, daß** das Verfahren mit Schnelltestverfahren Lateral-Flow-Test kombiniert wird und mindestens zwei, mehrere oder viele Symbole umfasst.

## Claims

1. A device for the allergy detection of molecules or molecule classes or molecule mixtures, **characterized in that**
a) at least two surface areas on a surface of the device are provided with immobilized molecules or molecule classes, wherein one surface area is used for control or standardization purposes, and the other surface area is used to detect an analyte, wherein
b) the two surface areas are structured in such a manner that they come in contact at the same time with an entire blood sample from which allergens are to be determined, and
c) wherein both surface areas are structured and arranged with respect to each other in such a manner that they are evaluated jointly and form an overall image of a graphical arrangement that can be read out visually, and
d) wherein the surface areas are disposed in a coordinate system or matrix and can be evaluated by the naked eye, and
e) wherein this device is designed as a vessel,
b) wherein the surface areas are represented in a plurality of symbols in a linear manner or in a matrix that is arranged in a different manner.

2. The device for the allergy detection of molecules or molecule classes or molecule mixtures according to claim 1, **characterized in that** the surface areas have a planar and/or spatial arrangement with respect to each other.

3. The device for the allergy detection of molecules or molecule classes or molecule mixtures according to claim 1 or 2, **characterized in that** the sample from which the analyte or analytes is/are to be determined is present in liquid, solid or gaseous form or in physical intermediate states or mixtures thereof.

4. A device for the allergy detection of molecules or molecule classes or molecule mixtures according to any one of claims 1 to 3, **characterized in that** the immobilized molecules or molecule classes are visually evaluated together by means of a detection reaction without additional technical aids, wherein the various surface areas appear colored, black or gray or in a mixture of colors and/or shades of gray.

5. A device for the allergy detection of molecules or molecule classes or molecule mixtures according to any one of claims 1 to 4, **characterized in that** the surface areas are located inside the vessel or one or more surface areas are located on the vessel wall.

6. A device for the allergy detection of molecules or molecule classes or molecule mixtures according to any one of claims 1 to 5, **characterized in that** the overall image of a graphical arrangement that can be read out visually is rendered visible as symbols, "-" for negative and "+" for positive, or a circle for negative and a circle with a dot or dots in it for positive.

7. A device for the allergy detection of molecules or molecule classes or molecule mixtures according to any one of claims 1 to 6, **characterized in that** the immobilized molecules or molecule classes and/or mixtures are selected from the group consisting of antibodies, antigens, allergens, DNA, RNA, enzymes, substrates, receptors, ligands, or combinations thereof.

8. A method for the allergy detection of molecules or molecule classes or molecule mixtures, comprising
a) establishing contact between a blood sample from which allergens are to be determined and a surface of a device in such a manner that they come in contact at the same time with the entire blood sample from which allergens are to be determined, wherein at least two surface areas on the surface of the device are provided with immobilized molecules or molecule classes and/or mixtures in such a way that one surface area is provided for control or standardization purposes, and the other is provided for the detection of an analyte, and wherein both surface areas are structured and arranged with respect to each other in such a manner that they are evaluated together and form an overall image of a graphic arrangement that can be read out visually, and
b) reading and evaluating the surface areas based on a coordinate system or matrix,
wherein this device is designed as a vessel and the surface areas are represented in a plurality of symbols in a linear manner or in a matrix that is arranged in a different manner.

9. The method according to claim 8, **characterized in that** the surface areas are read out in a planar and/or spatial manner.

10. The method according to either claim 8 or 9, **characterized in that** the various detection surface areas appear colored, black or gray or in a mixture of colors and/or shades of gray.

11. A method according to any one of claims 8 to 10, **characterized in that** the surface areas are read out in one or more symbols in a linear manner or in a matrix arranged in a different manner.

12. A method according to any one of claims 8 to 11, **characterized in that** the overall image of a graphical arrangement that can be read out visually is rendered visible as symbols, "-" for negative and "+" for positive, or a circle for negative and a circle with a dot or dots in it for positive.

13. The method according to claim 12, **characterized in that** symbols consisting of several circles inside each other having one center dot are rendered visible, said dot appearing only in a positive detection case, and wherein each individual circle only becomes visible above a certain concentration value of the analyte, or a star in which each of the star's points becomes visible above a certain concentration value and, in the positive case, a predefined point appears or the individual points detect the presence of several analytes and one point appears above a certain concentration value, or a combination of these symbols.

14. A method according to any one of claims 8 to 13, **characterized in that** the blood sample from which the analyte or analytes is/are determined is present in liquid, solid or gaseous form or in physical intermediate states or mixtures thereof.

15. A method according to any one of claims 8 to 14, **characterized in that** whole blood, capillary blood, umbilical cord blood, arterial or venous whole blood, blood serum or blood plasma are used as the blood sample.

16. A method according to any one of claims 8 to 15, **characterized in that** the blood sample is prepared before, during or in between by means of purification, aliquotation, derivatization and/or isolation in order to be applied onto the surface of the device according to the invention.

17. A method according to any one of claims 8 to 16, **characterized in that** the detection reactions of molecules or molecule classes or molecule mixtures are selected from dye, radio nucleotide, antibody, DNA or RNA, biotin, avidine or enzyme detection reactions or combinations thereof.

18. A method according to any one of claims 8 to 17, **characterized in that** the immobilized molecules or molecule classes and/or mixtures are visually determined by means of a detection reaction without additional technical aids.

19. A method according to any one of claims 8 to 18, **characterized in that** technical aids are employed for the read-out and/or evaluation in order to enable a visual evaluation, or the methods, for instance, densitometric methods, spectroscopic or electrochemical methods, are combined with the read-out and/or evaluation according to the invention.

20. A method according to any one of claims 8 to 19, **characterized in that** the method is combined with flow-through tests, agglutination tests and/or solid-phase tests and comprises one, more or many symbols.

21. A method according to any one of claims 8 to 20, **characterized in that** the method is combined with the fast lateral-flow test method and comprises at least two, more or many symbols.

## Revendications

1. Dispositif pour la détection de l'allergie à des molécules ou à des classes de molécules ou à des mélanges de molécules, **caractérisé en ce que** :
a) au moins deux aires portant des molécules ou classes de molécules immobilisées sont prévues sur une surface du dispositif, une aire étant utilisée pour le contrôle ou la normalisation et l'autre aire, pour la détection d'un analyte ;
b) dans lequel les deux aires sont configurées de manière que ces deux aires entrent en contact au même instant avec un échantillon de sang total dans lequel doivent être détectés des allergènes ; et
c) dans lequel les deux aires sont configurées et disposées relativement l'une à l'autre de manière que ces deux aires soient analysées ensemble et forment une image globale d'un agencement graphique pouvant être lu optiquement ; et
d) dans lequel les aires sont placées dans un système de coordonnées ou une matrice et peuvent être analysées à l'oeil nu ; et
e) dans lequel ce dispositif prend la forme d'un récipient ; et
f) dans lequel les aires sont représentées linéairement sous la forme d'une pluralité de symboles, ou dans une matrice organisée autrement.

2. Dispositif pour la détection de l'allergie à des molécules ou à des classes de molécules ou à des mélanges de molécules, selon la revendication 1, **caractérisé en ce que** les aires se trouvent l'une vis-à-vis de l'autre dans un agencement plan et/ou spatial.

3. Dispositif pour la détection de l'allergie à des molécules ou à des classes de molécules ou à des mélanges de molécules, selon l'une des revendications 1 ou 2, **caractérisé en ce que** l'échantillon dans lequel doivent être détectés le ou les analytes se présente sous une forme liquide, solide, gazeuse, ou dans des états physiques intermédiaires ou des mélanges de ceux-ci.

4. Dispositif pour la détection de l'allergie à des molécules ou à des classes de molécules ou à des mélanges de molécules, selon l'une des revendications 1 à 3, **caractérisé en ce que** les molécules ou classes de molécules immobilisées sont analysées ensemble visuellement sans autres accessoires techniques, à l'aide d'une réaction de détection, les différentes aires apparaissant en couleur, en noir ou en gris, ou sous la forme d'un mélange de couleurs et/ou de niveaux de gris.

5. Dispositif pour la détection de l'allergie à des molécules ou à des classes de molécules ou à des mélanges de molécules, selon l'une des revendications 1 à 4, **caractérisé en ce que** les aires se trouvent à l'intérieur du récipient ou **en ce qu'**une ou plusieurs aires se trouvent sur la paroi du récipient.

6. Dispositif pour la détection de l'allergie à des molécules ou à des classes de molécules ou à des mélanges de molécules, selon l'une des revendications 1 à 5, **caractérisé en ce que** l'image globale apparaît sous la forme d'un agencement graphique pouvant être lu optiquement et composé de symboles - pour négatif et + pour positif, ou cercle pour négatif et cercle avec un ou plusieurs points inscrits pour positif.

7. Dispositif pour la détection de l'allergie à des molécules ou à des classes de molécules ou à des mélanges de molécules, selon l'une des revendications 1 à 6, **caractérisé en ce que** les molécules ou classes de molécules et/ou mélanges qui sont immobilisés sont sélectionnés dans le groupe suivant : anticorps, antigènes, allergènes, ADN, ARN, enzymes, substrats, récepteurs, ligands et combinaisons de ceux-ci.

8. Procédé pour la détection de l'allergie à des molécules ou à des classes de molécules ou à des mélanges de molécules, comprenant :
a) l'opération consistant à mettre un échantillon sanguin dans lequel doivent être détectés des allergènes au contact d'un dispositif de manière que celui-ci entre en contact au même instant avec la totalité de l'échantillon sanguin dans lequel doivent être détectés des allergènes, où il est prévu sur la surface du dispositif au moins deux aires portant des molécules ou classes de molécules et/ou mélanges qui sont immobilisés, qui sont disposées de manière qu'une aire serve au contrôle ou à la normalisation, tandis que l'autre est prévue pour la détection d'un analyte, et où les deux aires sont configurées et disposées l'une par rapport à l'autre de manière que les deux aires soient analysées ensemble et forment une image globale d'un agencement graphique pouvant être lu optiquement ;
b) la lecture et l'analyse des aires à l'aide d'un système de coordonnées ou d'une matrice ;
ce dispositif prenant la forme d'un récipient, et les aires étant représentées sous la forme d'une pluralité de symboles, linéairement ou sous la forme d'une matrice organisée autrement.

9. Procédé selon la revendication 8, **caractérisé en ce que** la lecture des aires se fait de manière plane et/ou spatiale.

10. Procédé selon l'une des revendications 8 ou 9, **caractérisé en ce que** les différentes aires de détection apparaissent en couleur, en noir ou en gris, ou sous la forme d'un mélange de couleurs et/ou de niveaux de gris.

11. Procédé selon l'une des revendications 8 à 10, **caractérisé en ce que** les aires sont lues sous la forme d'un symbole ou d'une pluralité de symboles, linéairement ou sous la forme d'une matrice organisée autrement.

12. Procédé selon l'une des revendications 8 à 11, **caractérisé en ce que** l'image globale apparaît sous la forme d'un agencement graphique pouvant être lu optiquement et composé de symboles « - » pour négatif et « + » pour positif, ou cercle pour négatif et cercle avec un ou plusieurs points inscrits pour positif.

13. Procédé selon la revendication 12, **caractérisé en ce que** l'on utilise comme symboles :
plusieurs cercles concentriques avec un point central qui n'apparaît qu'en cas de détection positive et dans lequel chacun des cercles individuels n'apparaît qu'à partir d'une valeur définie de concentration de l'analyte ; ou une étoile dont chaque branche ne devient visible qu'à partir d'une valeur définie de concentration ou dont une branche définie au préalable apparaît dans le cas positif ou dont les différentes branches détectent différents analytes et dont une branche apparaît à partir d'une valeur définie de concentration, ou encore une combinaison de ces symboles.

14. Procédé selon l'une des revendications 8 à 13, **caractérisé en ce que** l'échantillon sanguin dans lequel doivent être détectés le ou les analytes se présente sous une forme liquide, solide, gazeuse, ou dans des états physiques intermédiaires ou des mélanges de ceux-ci.

15. Procédé selon l'une des revendications 8 à 14, **caractérisé en ce que** l'échantillon sanguin utilisé est du sang complet, du sang capillaire, du sang de cordon ombilical, du sang complet artériel ou veineux, du sérum sanguin ou du plasma sanguin.

16. Procédé selon l'une des revendications 8 à 15, **caractérisé en ce que** l'échantillon sanguin est préparé avant, pendant ou entre-temps par épuration, aliquotation, transformation en dérivé et/ou isolement pour son application à la surface du dispositif selon l'invention.

17. Procédé selon l'une des revendications 8 à 16, **caractérisé en ce que** les réactions de détection de molécules ou de classes de molécules ou de mélanges de molécules sont choisies parmi les réactions de détection par colorants, radionucléotides, anticorps, ADN ou ARN, biotine, avidine ou enzyme, ou des combinaisons de celles-ci.

18. Procédé selon l'une des revendications 8 à 17, **caractérisé en ce que** les molécules ou classes de molécules et/ou mélanges qui sont immobilisés sont détectés visuellement sans autres accessoires techniques, à l'aide d'une réaction de détection.

19. Procédé selon l'une des revendications 8 à 18, **caractérisé en ce que** l'on utilise pour la lecture et/ou l'analyse des accessoires techniques pour permettre une analyse visuelle ou **en ce que** le procédé est combiné avec la lecture et/ou l'analyse selon l'invention, par exemple procédé densitométrique, procédé spectroscopique ou électrochimique.

20. Procédé selon l'une des revendications 8 à 19, **caractérisé en ce que** le procédé est combiné avec des essais en écoulement continu, des essais d'agglutination et/ou des essais en phase solide et comprend un symbole, plusieurs symboles ou de nombreux symboles.

21. Procédé selon l'une des revendications 8 à 20, **caractérisé en ce que** le procédé est combiné avec le procédé d'essai rapide à savoir l'essai à écoulement latéral et comprend deux symboles, plusieurs symboles ou de nombreux symboles.
